# EUROPÄISCHE PATENTANMELDUNG

(11) **EP 2 360 159 A1**
(43) Veröffentlichungstag der Anmeldung: **24.08.2011**
(21) Anmeldenummer: 10153348.7
(22) Anmeldetag: 11.02.2010
(51) Int. Cl.: C07D 495/04, A61P 7/02, A61K 31/4365, A61K 9/20, A61K 9/14, A61K 9/16

(54) **Prasugrel in mikronisierter, kristalliner Form und pharmazeutische Zusammensetzung davon**

(71) Anmelder: Ratiopharm GmbH, 89079 Ulm (DE)
(72) Erfinder: BRUECK, Sandra, 85570, Ottenhofen (DE); PAETZ, Jana, 82272, Moorenweis (DE)
(74) Vertreter: Teipel, Stephan

(57) **Zusammenfassung**

Die vorliegende Erfindung betrifft Prasugrel oder ein pharmazeutisch verträgliches Salz davon, Zusammensetzungen, die diesen Wirkstoff enthalten sowie pharmazeutische Zusammensetzungen, die diesen Wirkstoff oder eine diesen Wirkstoff enthaltende Zusammensetzung enthalten. Die vorliegende Erfindung betrifft ferner Verfahren zur Herstellung der neuen Zusammensetzungen.

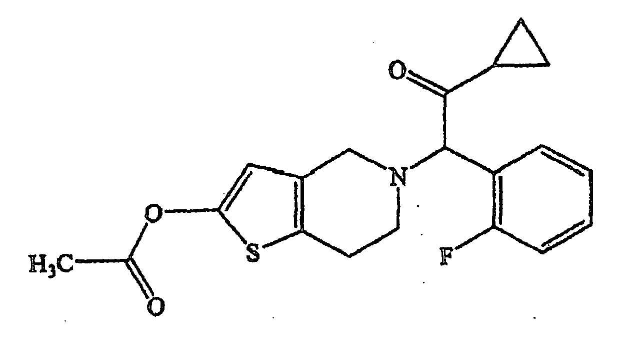

## Beschreibung

Die vorliegende Erfindung betrifft Prasugrel oder ein pharmazeutisch verträgliches Salz davon, Zusammensetzungen, die diesen Wirkstoff enthalten sowie pharmazeutische Zusammensetzungen, die diesen Wirkstoff oder eine diesen Wirkstoff enthaltende Zusammensetzung enthalten. Die vorliegende Erfindung betrifft ferner Verfahren zur Herstellung der neuen Zusammensetzungen.

Prasugrel hat den chemischen Namen 2-Acetoxy-5-(a-cyclopropylcarbonyl-2-fluorbenzyl)-4,5,6,7-tetrahydrothieno[3,2-c]pyridin. Prasugrel hat die folgende Strukturformel:

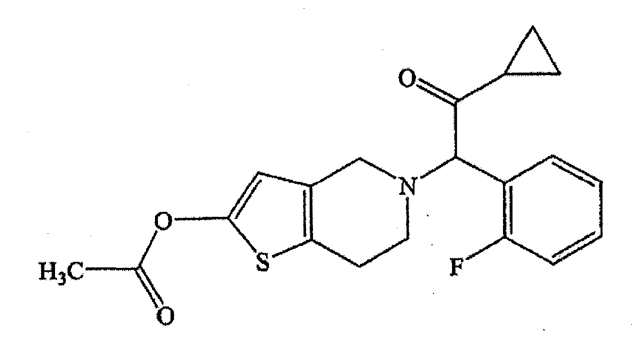

Ein Verfahren zur Herstellung von kristallinem Prasugrel wird in der EP-A-0 542 411 offenbart.

Verschiedene Säureadditionssalze von Prasugrel sind in der EP-A-1 298 132 offenbart. Hierbei handelt es sich ebenfalls um kristalline Salze.

Prasugrel wird als Plättchenaggregationshemmer oral verabreicht. Problematisch hierbei ist, dass es sich bei Prasugrel um einen schwer löslichen Wirkstoff handelt. Die freie Base des Wirkstoffs weist eine Löslichkeit von 57 µg/ml in Wasser auf, aber auch die Salze zeigen nur eine begrenzte Löslichkeit.

Die Löslichkeit eines Wirkstoffs lässt sich oftmals durch Mikronisation der Wirkstoffteilchen erhöhen. Aufgrund der starken Oxidationsempfindlichkeit von Prasugrel ist eine direkte Trockenvermahlung im µm-Bereich jedoch nicht möglich, da hierbei bedingt sowohl durch den mechanischen als auch thermischen Einfluss kombiniert mit der Oberflächenvergrößerung der chemische Abbau zu hoch ist.

Darüber hinaus weist Prasugrel auch einen stark pH-abhängigen Abbau auf. Bei pH-Werten von < 3 ist ein Abbau von mehr als 1 % nach bereits 2 Stunden zu beobachten. Dies schränkt die möglichen Hilfsstoffe bei der Herstellung pharmazeutischer Formulierungen ein.

Im Stand der Technik wurden verschiedene Vorschläge gemacht, die Löslichkeit von Prasugrel aus pharmazeutischen Zusammensetzungen zu verbessern und die Lagerstabilität entsprechender pharmazeutischer Zusammensetzungen zu erhöhen. So schlägt die WO 2008/072535 vor, Prasugrel zusammen mit einer niedrig substituierten Hydroxypropylcellulose zu pharmazeutischen Zusammensetzungen zu verarbeiten. Hierzu wird der Wirkstoff mit niedrig substituierter Hydroxypropylcellulose, Hydroxypropylcellulose und Lactose für 3 Minuten intensiv gemischt. Der erhaltenen Mischung wird Magnesiumstearat zugegeben und die Mischung wird erneut gemischt. Das erhaltene Pulver wird zu Tabletten verpresst. Prasugrel und Hydroxypropylcellulose werden hierbei im Verhältnis von etwa 1:1 gemischt. Über eine Mikronisierung des Wirkstoffs während des Mischungsprozesses wird nicht berichtet.

Ähnliche pharmazeutische Zusammensetzungen, die neben Prasugrel wasserlösliche Polymere wie Hydroxypropylcellulose, Hydroxypropylmethylcellulose und Natriumcarboxymethylcellulose enthalten, sind in WO 2008/069262, WO 2008/072532 und WO 2008/072534 offenbart.

Die WO 2008/072533 offenbart Prasugrel-haltige pharmazeutische Zusamensetzungen, die neben dem Wirkstoff Hydroxypropylcellulose in einem Gewichtsverhältnis von etwa 1:1,5 enthalten. Die Mischungen werden dabei für 3 Minuten unter hohem Energieeintrag in einem Henschel-Mischer gemischt. Im angegebenen Vergleichsbeispiel wird eine entsprechende Mischung für 30 Minuten in einem Diffusionsmischer gemischt. Auch hier erfolgt keine Mikronisierung des Wirkstoffs.

Um die Lagerstabilität Prasugrel-haltiger Tabletten zu erhöhen, schlägt die WO 2008/073759 vor, diese in einem luft- und feuchtigkeitsundurchlässigen Behälter unter einem positiven Flüssiggasdruck zu verpacken.

Es besteht somit weiterhin ein Bedürfnis nach Prasugrel-haltigen Formulierungen, die den Wirkstoff in einer möglichst löslichen Form zur Verfügung stellen. Dabei besteht auch ein Bedürfnis nach einer Verbesserung der Lösungsgeschwindigkeit sowie der Bioverfügbarkeit des Wirkstoffs. Außerdem ist es wünschenswert, die chemische Stabilität des Wirkstoffs zu verbessern, insbesondere gegen oxidativen und hydrolytischen Abbau sowie bei thermischer Belastung. Ein weiteres Problem besteht in der Verbesserung der Verarbeitbarkeit von Prasugrel zu pharmazeutischen Zusammensetzungen, die Verringerung der Hygroskopie der Zusammensetzung, sowie die Verbesserung der Lagerstabilität entsprechender Zusammensetzungen.

Es wurde nun überraschend gefunden, dass Prasugrel unter bestimmten Verarbeitungsbedingungen in eine mikronisierte, kristalline Form überführt werden kann, wobei diese mikronisierte, kristalline Form bzw. Zusammensetzungen, die diese Form enthalten, eines oder mehrere der vorstehend genannten Probleme lösen.

Die vorliegende Erfindung betrifft somit Prasugrel oder ein pharmazeutisch verträgliches Salz davon in mikronisierter, kristalliner Form.

Unter kristalliner Form wird vorliegend jede Form des Wirkstoffs oder seines pharmazeutisch verträglichen Salzes verstanden, die im Wesentlichen frei und vorzugsweise ganz frei von amorphen Anteilen des Wirkstoffs oder seines pharmazeutisch verträglichen Salzes ist. Unter im Wesentlichen frei von kristallinen Anteilen wird dabei verstanden, dass die Form weniger als 10 Gew.-%, vorzugsweise weniger als 5 Gew.-% und am bevorzugtesten weniger als 1 Gew.-% Wirkstoff in amorpher Form enthält, wobei sich die Gewichtsprozentangaben auf die Gesamtmenge von Prasugrel oder einem pharmazeutisch verträglichen Salz davon beziehen.

Kristalline Anteile eines vorliegenden Wirkstoffs können beispielsweise durch DSC-Messung und IR-Spektroskopie charakterisiert werden.

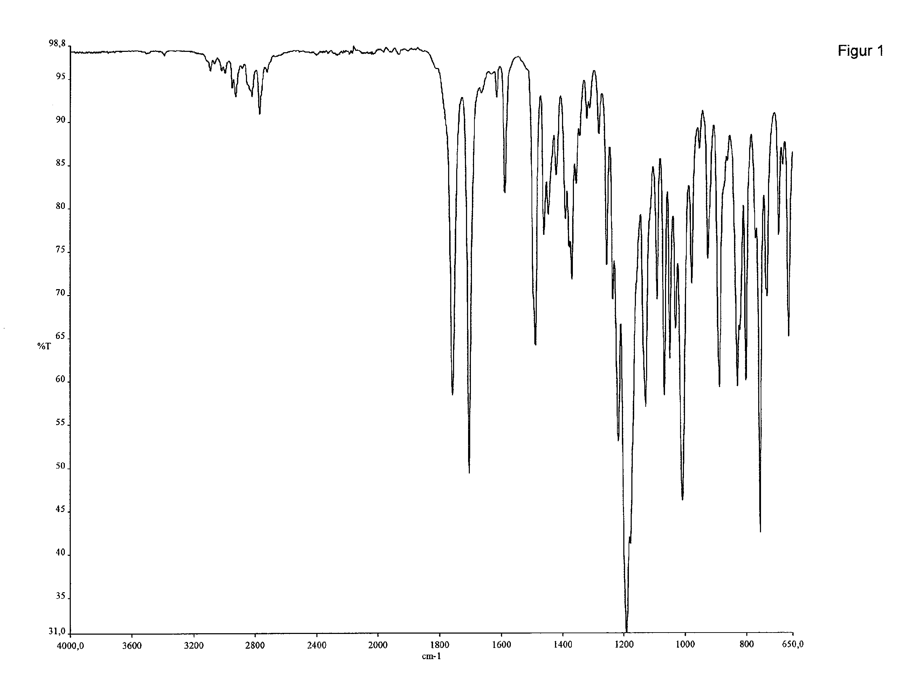

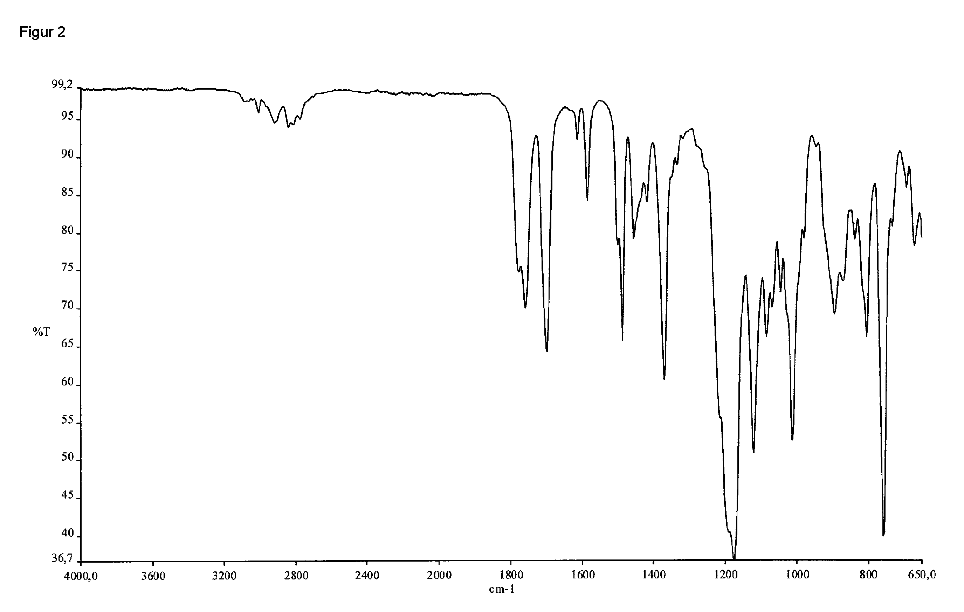

Die kristalline Form kann von der nicht-kristallinen Form des Wirkstoffs beispielsweise auch durch IR-Spektroskopie unterschieden werden. Figur 1 zeigt das IR-Spektrum von kristallinem Prasugrel. Figur 2 zeigt das IR-Spektrum von amorphem Prasugrel. Außer einem Shift der Peaks erkennt man für den amorphen Wirkstoff einen zusätzlichen Peak bei etwa 1778 cm⁻¹ und für das kristalline Prasugrel zusätzlich Peaks bei etwa 1254 cm⁻¹ und etwa 830 cm⁻¹. Die Banden der IR-Spektren sind zudem in der folgenden Tabelle 1 (zu Figur 1) und 2 (zu Figur 2) zusammengefasst.

**Tabelle 1**

| Wellenzahl | **Transmission** | Wellenzahl | **Transmission** | Wellenzahl | **Transmission** | Welienzahl | **Transmission** | Wellenzahl | **Transmission** |
|---|---|---|---|---|---|---|---|---|---|
| [cm⁻1] | **[%]** | [cm⁻1] | **[%]** | [cm⁻1] | **[%]** | [cm⁻1] | **[%]** | [CM⁻1] | **[%]** |
| **2922.8** | 92.9 | **2817.9** | 93.0 | **2768.0** | 90.9 | **1756.3** | 58.3 | **1702.5** | 49.2 |
| **1612.8** | 92.8 | **1586.1** | 81.7 | **1487.3** | 64.1 | **1459.0** | 76.9 | **1444.4** | 79.3 |
| **1419.0** | 83.9 | **1388.6** | 78.7 | **1367.5** | 71.7 | **1353.2** | 82.9 | **1318.8** | 90.4 |
| **1279.2** | 88.6 | **1253.6** | 73.4 | **1233.4** | 69.3 | **1216.3** | 52.9 | **1191.5** | 30.9 |
| **1127.1** | 57.0 | **1090.0** | 69.5 | **1066.6** | 58.3 | **1049.0** | 62.6 | **1030.2** | 66.1 |
| **1008.7** | 46.1 | **978.1** | 71.2 | **952.7** | 86.9 | **925.8** | 74.2 | **887.7** | 59.3 |
| **829.5** | 59.3 | **801.6** | 59.9 | **757.4** | 42.2 | **733.1** | 69.8 | **694.9** | 76.9 |
| **662.5** | 65.0 | | | | | | | | |

**Tabelle 2**

| **Wellenzahl** | Transmission | **Wellenzahl** | Transmission | **Wellenzahl** | Transmission | **Wellenzahl** | Transmission | **Wellenzahl** | Transmission |
|---|---|---|---|---|---|---|---|---|---|
| **[cm⁻1]** | [%] | **[cm⁻1]** | [%] | **[cm⁻1]** | [%] | **[cm⁻1]** | [%] | **[cm⁻1]** | [%] |
| **2917.1** | 94.4 | **2841.6** | 93.8 | **1777.9** | 74.7 | **1758.6** | 70.0 | **1698.3** | 64.2 |
| **1612.7** | 92.2 | **1584.8** | 84.2 | **1487.3** | 65.6 | **1455.6** | 79.1 | **1418.4** | 84.1 |
| **1370.2** | 60.6 | **1176.8** | 36.7 | **1122.0** | 50.9 | **1085.3** | 66.2 | **1046.0** | 72.0 |
| **1012.9** | 52.5 | **895.4** | 69.1 | **838.1** | 79.1 | **805.2** | 66.2 | **758.0** | 39.8 |
| **693.7** | 85.8 | **671.0** | 78.2 | | | | | | |

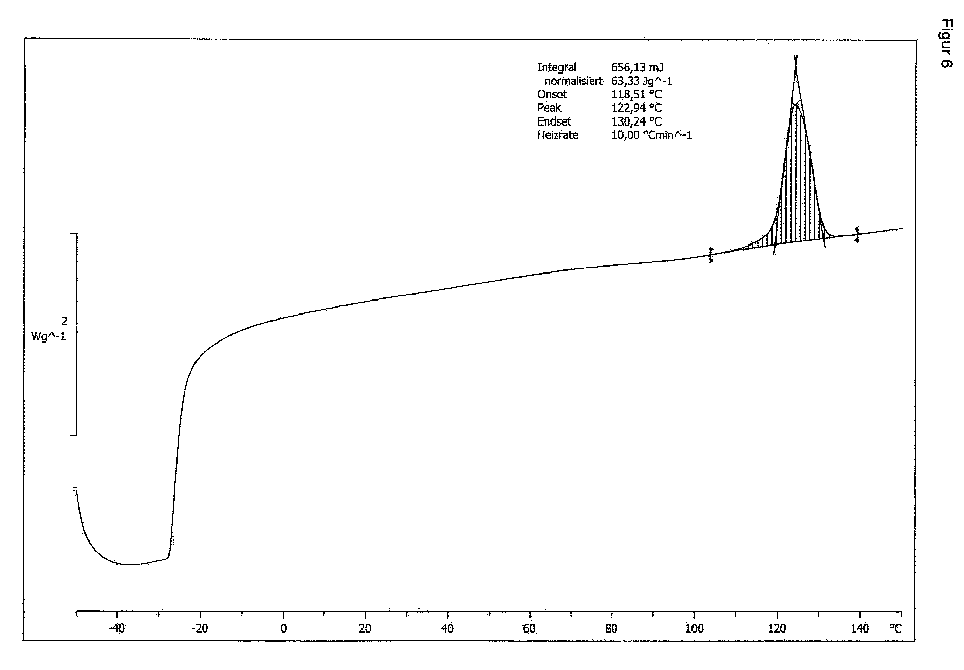

Ferner zeigt die kristalline Base im DSC einen scharfen Peak bei 123 °C. Ein Beispiel hierfür ist in Figur 6 dargestellt.

Vorliegend wird unter "Wirkstoff' Prasugrel oder ein pharmazeutisch verträgliches Salz davon verstanden. Als pharmazeutisch verträgliche Salze eignen sich beispielsweise das Hydrochlorid, das Hydrobromid, das Sulfat, das Phosphat, Alkylsulfonsäuresalze, wie Methansulfonat, Trifluormethansulfonat und Ethansulfonat, Arylsulfonsäuresalze, wie Benzolsulfonat, p-Toluolsulfonat, 1-Naphthalinsulfonat, 2-Naphthalinsulfonat und 1,5-Naphthalindisulfonat, sowie Salze organischer Säuren, wie Acetat, Malat, Fumarat, Succinat, Citrat, Ascorbat, Tartrat, Oxalat und Maleat. Vorzugsweise handelt es sich bei dem Wirkstoff gemäß der vorliegenden Erfindung um Prasugrel-Base.

Erfindungsgemäß wurde überraschend gefunden, dass Prasugrel oder ein pharmazeutisch verträgliches Salz davon in mikronisierter, kristalliner Form durch bestimmte Herstellungsverfahren gewonnen werden kann. Beispielsweise kann der Wirkstoff zusammen mit einem hydrophilen Polymer in einem Vermahlungsprozess verarbeitet und dadurch mikronisiert werden. Es wird vermutet, dass die Gegenwart des hydrophilen Polymers den Abbau des Wirkstoffs während des Vermahlens reduziert. Der Wirkstoff kann zusammen mit dem hydrophilen Polymer beispielsweise durch ein Trockenvermahlungsverfahren oder durch eine Nassvermahlung mikronisiert werden. In beiden Verfahren hat es sich als wichtig erwiesen, dass nur geringe Mengen Polymer vorhanden sind, um den kristallinen Zustand des Wirkstoffs zu stabilisieren und eine Amorphisierung zu verhindern. Außerdem müssen Wirkstoff und Polymer innig miteinander vermischt werden, damit der Wirkstoff in der mikronisierten Form nicht wieder zu größeren Wirkstoffpartikeln agglomeriert. Geeignete Herstellungsverfahren werden nachfolgend noch näher beschrieben.

Um eine Polymermenge zur Verfügung zu stellen, die ausreicht, die Zersetzung des Wirkstoffs zu reduzieren und gleichzeitig die Agglomeration der mikronisierten Wirkstoffpartikel zu verhindern, sollte das Gewichtsverhältnis von Prasugrel, bezogen auf die freie Base, zu hydrophilem Polymer > 1:4, vorzugsweise > 1:1, vorzugsweise ≥ 2:1, mehr bevorzugt ≥ 5:1 sein. Bevorzugte Gewichtsverhältnisse von Prasugrel zu hydrophilem Polymer betragen beispielsweise etwa 4:1 oder etwa 5:1. Wird bei der Vermahlung mehr Polymer zugesetzt, beispielsweise in einem Verhältnis von Wirkstoff zu hydrophilem Polymer von < 1:4, so erhält man eine Zusammensetzung, in der der Wirkstoff amorph vorliegt.

### Polymer:

Als hydrophile Polymere eignen sich wasserlösliche Polymere, beispielsweise Polymere, die bei Raumtemperatur eine Wasserlöslichkeit von > 0,01 mg/ml aufweisen. Es können ein oder mehrere hydrophile Polymere zusammen mit dem Wirkstoff zu den erfindungsgemäßen Zusammensetzungen verarbeitet werden. Außerdem können die Zusammensetzungen wenn gewünscht weitere pharmazeutisch verträgliche Hilfsstoffe enthalten.

Generell umfasst die Bezeichnung "hydrophiles Polymer" Polymere mit polaren Gruppen. Beispiele für polare Gruppen sind Hydroxy, Amino, Carboxy, Carbonyl, Ether, Ester, Sulfonate. Hydroxygruppen sind besonders bevorzugt.

Das hydrophile Polymer hat üblicherweise ein Molekulargewicht im Bereich zwischen 1000 und 250.000 g/mol, bevorzugt, 2000 bis 100.000 g/mol, und besonders bevorzugt zwischen 4000 und 50.000 g/mol. Ferner hat eine 2 Gew.-%ige Lösung des hydrophilen Polymers in reinem Wasser bevorzugte eine Viskosität zwischen 2 und 8 mPas bei 25°C. Die Viskosität wird bestimmt nach der Europäischen Pharmacopoeia (Ph. Eur.), 6. Auflage, Abschnitt 2.2.10.

Ferner hat das hydrophile Polymer bevorzugt eine Glas-Übergangstemperatur (Tg) zwischen 20°C und 150°C, bevorzugt 25°C bis 100°C. Die Glas-Übergangstemperatur (Tg) ist die Temperatur, bei der das hydrophile Polymer beim Abkühlen spröde wird und weich beim Erwärmen. Das bedeutet, dass das hydrophile Polymer oberhalb der Glas-Übergangstemperatur weich wird und sich plastisch verformen lässt ohne zu brechen. Die Glas-Übergangstemperatur wird mittels einer Mettler-Toledo^{®} DSC 1 bestimmt, wobei eine Erwärmungsrate von 10°C/min und eine Abkühlrate von 15°C/min verwendet wird.

Beispielsweise kann das hydrophile Polymer ausgewählt sein aus der Gruppe, bestehend aus Cellulosederivaten, wie Hydroxypropylmethylcellulose, Hydroxypropylcellulose, Carboxymethylcellulose, bevorzugt als Natrium- oder Calciumsalz, Hydroxyethylcellulose, Polyvinylpyrrolidon, bevorzugt mit einem Molekulargewicht von 10.000 bis 60.000 g/ml, Copolymeren von Polyvinylpyrrolidon, bevorzugt Copolymere umfassend Vinylpyrrolidon- und Vinylacetateinheiten (z.B. Povidon, VA64, BASF), bevorzugt mit einem Molekulargewicht zwischen 40.000 und 70.000 g/ml, Polyoxyethylen-Alkylether, Polyethylenglycol, Co-Block-Polymeren von Ethylenoxid und Propylenoxid (Poloxamer, Pluronic), Polymethacrylatederivaten, Polyvinylalkohol, Polyvinylalkoholderivaten und Polyethylenglycolderivaten, wie Macrogolglycerinstearat. Bevorzugte hydrophile Polymere sind Macrogolglycerinstearat, Blockcopolymere aus Ethylenoxid und Propylenoxid, Polyethylenglycol und Hydroxypropylmethylcellulose.

### Mikronisierung/Partikelgröβenverteilung:

Der Wirkstoff Prasugrel oder ein pharmazeutisch verträgliches Salz davon liegen in mikronisierter Form in der pharmazeutischen Zusammensetzung vor. Das bedeutet, dass der Volumen-gewichtete mittlerer Teilchendurchmesser D50 zwischen 0,1 und 10 µm, vorzugsweise zwischen 0,1 und 5 µm liegt und der D90 zwischen 0,2 und 20 µm, vorzugsweise zwischen 0,2 und 16 µm.

Die Bestimmung der Partikelgröße wird gemäß Europäischen Pharmacopoeia (Ph. Eur.), 6. Auflage, Abschnitt 2.9.31 vorzugsweise mit einem Mastersizer 2000 von Malvern Instruments durchgeführt. Die Auswertung erfolgt nach dem Fraunhofer-Modell.

### Vermahlungsprozess:

Die mikronisierte, kristalline Form des Wirkstoffs kann durch einen Vermahlungsprozess in Gegenwart des hydrophilen Polymers erhalten werden. Bei der Vermahlung kann es sich um eine Trocken- oder Nassvermahlung handeln. An den Vermahlungsschritt können sich weitere Verarbeitungsschritte, wie beispielsweise eine Lyophilisierung, Sprühtrocknung oder eine Granulation auf einen Träger anschließen.

Neben dem vorstehend bereits beschriebenen Gewichtsverhältnis von Wirkstoff zu hydrophilem Polymer ist eine ausreichend lange Vermahlung des Wirkstoff-Polymer-Gemisches notwendig, um den Wirkstoff zu mikronisieren, ohne ihn dabei in die amorphe Form zu überführen. Eine geeignete Zeitdauer kann vom Fachmann daran bestimmt werden, ob der eingesetzte kristalline Wirkstoff nach dem Vermahlungsschritt in der gewünschten Partikelgrößenverteilung vorliegt. Gegebenenfalls muss der Zeitraum des Vermahlungsschritts verlängert werden. Eine Vermahlung von mindestens 30 min, vorzugsweise mindestens 1 h führt in der Regel zu der gewünschten Mikronisierung des Wirkstoffs.

Die Nassvermahlung kann z.B. in einem Ultraturrax durchgeführt werden bei Umdrehungen zwischen 11000 und 24000 Umdrehungen pro Minute, vorzugsweise etwa 11.000 Umdrehungen pro Minute.

Alternativ kann die Nassvermahlung auch in einer Netsch-Mühle (z.B. Netsch Micro Cer) durchgeführt werden. Hierbei kann eine besonders feine und enge Partikelgrößenverteilung erreicht werden.

Wenn es sich bei dem Vermahlungsschritt um eine Trockenvermahlung handelt, so erfolgt diese beispielsweise in einer Kugelmühle, gegebenenfalls unter Kühlung, beispielsweise mittels flüssigem Stickstoff.

Besonders bevorzugt eignet sich für die Trockenvermahlung eine Luftstrahlmühle wie z.B. eine 50 AS der Firma Hosokava Alpine, wobei der Druck des Injektorgases zischen 2 und 8 bar, vorzugsweise bei etwa 5 bar liegt und das Mahlgas einen Druck zwischen 0.5 und 5 bar, vorzugsweise etwa 2 bar besitzt.

Die sich an die Nassvermahlung anschließende Sprühtrocknung kann z.B. in einem Büchi B290 durchgeführt werden.

Neben dem einen oder den mehreren hydrophilen Polymeren kann die erfindungsgemäße Zusammensetzung bei ihrer Herstellung durch Vermahlung weitere pharmazeutisch verträgliche Hilfsstoffe enthalten. Besonders geeignet sind beispielsweise Emulgatoren, insbesondere mit einem HLB-Wert > 12. Ganz besonders geeignet ist hierfür SDS (Natriumlaurylsulfat). Menge und Art der eingesetzten hydrophilen Polymere und sonstigen Hilfsstoffe haben einen Einfluss auf die Freisetzung und Stabilisierung der mikronisierten, kristallinen Form des Wirkstoffs.

Die vorliegende Erfindung betrifft somit auch ein Verfahren zur Herstellung einer vorstehend beschriebenen Zusammensetzung, umfassend eine Trocken- oder Nassvermahlung von Prasugrel oder einem pharmazeutisch verträglichen Salz davon in Gegenwart des hydrophilen Polymers. Wenn es sich bei dem Vermahlungsschritt um eine Trockenvermahlung handelt, so kann diese unter Kühlung erfolgen, beispielsweise mittels flüssigem Stickstoff. In einer bevorzugten Ausführung erfolgt die Vermahlung durch eine Luftsstrahlmühle.

Die vorliegende Erfindung betrifft darüber hinaus eine durch das vorstehend beschriebene Verfahren erhältliche Zusammensetzung.

### Pharmazeutische Zusammensetzung:

Der vorstehend beschriebene Wirkstoff in mikronisierter, kristalliner Form bzw. die vorstehend beschriebenen Zusammensetzungen können anschließend durch Verwendung weiterer pharmazeutisch verträglicher Hilfsstoffe zu pharmazeutischen Zusammensetzungen, insbesondere zur Plättchenaggregationshemmung weiterverarbeitet werden. Bei den fertigen Darreichungsformen kann es sich z.B. um Tabletten, Kapseln, Sachets oder Pulver handeln.

Die pharmazeutische Zusammensetzung kann zusätzlich zum hydrophilen Polymer einen oder mehrere weitere pharmazeutisch akzeptable Hilfsstoffe enthalten, wie z.B. Füllmittel, Gleitmittel, Fließregulierungsmittel, Trennmittel, Sprengmittel (Lexikon der Hilfsstoffe für Pharmazie, Kosmetik und angrenzende Gebiete", herausgegeben von H. P. Fiedler, 4. Auflage, und "Handbook of Pharmaceutical Excipients", 3. Auflage, herausgegeben von Arthur H. Kibbe, American Pharmaceutical Association, Washington, USA und Pharmaceutical Press, London).

Füllmittel: Die pharmazeutische Zusammensetzung kann ein oder mehrere Füllmittel enthalten. Im Allgemeinen handelt es sich bei einem Füllmittel um eine Substanz die das Bulk-Volumen der Mischung und somit die Größe der resultierenden Arzneiform vergrößert. Bevorzugte Beispiele für Füllmittel sind Lactose, mikrokristalline Cellulose (z.B. Avicel) und Calciumhydrogenphosphat. Das Füllmittel kann einen Anteil von 0 bis 90 Gew.-%, bevorzugt zwischen 25 und 85 Gew.-% des Gesamtgewichts der Zusammensetzung haben.

Gleitmittel: Die Funktion des Gleitmittels ist es, sicherzustellen, dass die Tablettenpressung und der Auswurf ohne große Reibung zwischen den Festoffen und den Wandungen erfolgt. Das Gleitmittel ist vorzugsweise ein Erdalkalimetallstearat, wie Magnesiumstearat oder eine Fettsäure, wie Stearinsäure. Das Gleitmittel ist üblicherweise in einer Menge von 0 bis 2 Gew.-%, bevorzugt zwischen 0,5 und 1,5 Gew.-% des Gesamtgewichts der pharmazeutischen Zusammensetzung vorhanden.

Sprengmittel: Gewöhnlich wird unter einem Sprengmittel eine Substanz verstanden, die in der Lage ist, die Tablette in kleinere Teile aufzubrechen, sobald sie in Kontakt mit einer Flüssigkeit ist. Bevorzugte Sprengmittel sind Croscarmellose Natrium, Natriumcarboxymethylstärke, quervernetztes Polyvinylpyrrolidon (Crospovidon) oder Natriumcarboxymethylglycolat (z.B. Explotab) und Natriumbicarbonat. Das Sprengmittel ist normalerweise in einem Anteil von 0 bis 20 Gew.-%, bevorzugt zwischen 1 und 15 Gew.-% des Gesamtgewichts der Zusammensetzung vorhanden.

Fließregulierungsmittel: Als Fließregulierungsmittel kann z.B. kolloidales Siliziumdioxid verwendet werden. Bevorzugt ist das Fließregulierungsmittel in einer Menge von 0 bis 8 Gew.-%, mehr bevorzugt in einer Menge zwischen 0.1 und 3 Gew.-% des Gesamtgewichts der Zusammensetzung vorhanden.

Stabilisatoren/Antioxidantien: Als Stabilisatoren und Antioxidantien können Ascorbinsäure und Acorbate, EDTA und seine Salze, Citronensäure und Citrate, Butylhydroxyanisol, Butylhydroxytoluol oder Vitamin E verwendet werden. Bevorzugt wird Vitamin E als Stabilisator verwendet.

In den anliegenden Figuren zeigen:
Figur 1 das IR-Spektrum von kristallinem Prasugrel,
Figur 2 das IR-Spektrum von amorphem Prasugrel,
Figur 3 das Freisetzungsprofil der Formulierung aus Beispiel 1 (Dreiecke) im Vergleich zu Freisetzungsprofil des kommerziellen Prasugrel-Produkts Efient^{®} (Vierecke),
Figur 4 das Freisetzungsprofil der Formulierung aus Beispiel 2 (Dreiecke) im Vergleich zu Freisetzungsprofil des kommerziellen Prasugrel-Produkts Efient^{®} (Vierecke),
Figur 5 das Freisetzungsprofil der Formulierung aus Beispiel 3 (Dreiecke) im Vergleich zu Freisetzungsprofil des kommerziellen Prasugrel-Produkts Efient^{®} (Vierecke),
Figur 6 das DSC der vermahlenen Zusammensetzung aus Beispiel 3,
Figur 7 das Freisetzungsprofil der Formulierung aus Beispiel 4 (Dreiecke) im Vergleich zu Freisetzungsprofil des kommerziellen Prasugrel-Produkts Efient^{®} (Vierecke),
Figur 8 das Freisetzungsprofil der Formulierung aus Beispiel 5 (Dreiecke) im Vergleich zu Freisetzungsprofil des kommerziellen Prasugrel-Produkts Efient^{®} (Vierecke),
Figur 9 das Freisetzungsprofil der Formulierung aus Beispiel 6 (Dreiecke) im Vergleich zu Freisetzungsprofil des kommerziellen Prasugrel-Produkts Efient^{®} (Vierecke),
Figur 10 das Freisetzungsprofil der Formulierung aus Beispiel 7 (Dreiecke) im Vergleich zu Freisetzungsprofil des kommerziellen Prasugrel-Produkts Efient^{®} (Vierecke),
Figur 11 das Freisetzungsprofil der Formulierung aus Beispiel 8 (Dreiecke) im Vergleich zu Freisetzungsprofil des kommerziellen Prasugrel-Produkts Efient^{®} (Vierecke).

Die vorliegende Erfindung wird anhand der nachfolgenden Beispiele näher erläutert, ohne dass diese einschränkend ausgelegt werden sollen.

Neben dem Wirkstoff werden folgende Verbindungen in den Beispielen eingesetzt:

| | |
|---|---|
| Kollidon VA 64: | Copolyvinylpyrrolidon |
| Pluronic: | Blockcopolymere aus Ethylenoxid und Propylenoxid |
| Avicel: | mikrokristalline Cellulose |
| HPMC: | Hydroxypropylmethylcellulose |
| Kollidon: | Polyvinylpyrrolidon |
| SDS: | Natriumlaurylsulfat |
| Acdisol: | Natrium-Croscarmellose |

Allgemeine Verfahren zur Herstellung der der Formulierungen

### Vermahlung mit Ultraturrax (Nassvermahlung)

Bei allen Vermahlungsversuchen mit dem Ultraturrax T25 wird der Wirkstoff, das Polymer und gegebenenfalls das SDS in Wasser 1h bei 11000 U/min dispergiert. Danach wird diese Suspension sprühgetrocknet (Büchi B290; Einstellungen = Aspirator 70, Zuluft 100°C). Das erhaltene Intermediat wird dann für 24h bei 30°C nachgetrocknet Die restlichen Hilfsstoffe werden zugegeben, die Gesamtmischung über ein 500 µm Sieb gegeben, im Freifallmischer gemischt, und zu Tabletten verpresst. Alternativ können bei der Nassvermahlung auch die Planetenkugelmühle PM100 von Retsch, von Netsch die MicroCer, und von Fryma die Koruma Coball Mill MS12 verwendet werden.

### Vermahlung mit der Luftstrahlmühle 50AS (Trockenvermahlung)

Auch hier wird der Wirkstoff, das Polymer und gegebenenfalls das SDS gemeinsam über die Mühle gegeben (Luftstrahlmühle; Einstellungen: Mahlgas = 2 bar, Injektorgas= 5 bar). Nach dem Mahlen werden die restlichen Hilfsstoffe zugegeben, über ein 500 µm Sieb gegeben, im Freifallmischer gemischt und zu Tabletten verpresst.

### Beispiel 1 (Formulierung 1):

| **Inhaltsstoff** | **mg/Tablette** | **Gew.-%** |
|---|---|---|
| Prasugrel Base | 5,00 | 4,23 |
| HPMC 603 | 1,00 | 0,85 |
| Pluronic 127 | 0,25 | 0,21 |
| Acdisol | 12,00 | 10,15 |
| Avicel PH 101 | 100, 00 | 84,57 |
| **Gesamtgewicht** | **118,25** | **100,00** |

### Vermahlung im Ultraturrax (Nassvermahlung)

### Beispiel 2 (Formulierung 2)

| **Inhaltsstoff** | **mg/Tablette** | **Gew.-%** |
|---|---|---|
| Prasugrel Base | 5, 00 | 4,23 |
| HPMC 603 | 1,00 | 0,85 |
| SDS | 0,25 | 0,21 |
| Acdisol | 12,00 | 10,15 |
| Avicel PH 101 | 100, 00 | 84,57 |
| **Gesamtgewicht** | **118,25** | **100,00** |

### Vermahlung im Ultraturrax (Nassvermahlung)

### Beispiel 2a (Formulierung 2a)

| **Inhaltsstoff** | **mg/Tablette** | **Gew.-%** |
|---|---|---|
| Prasugrel Base | 5,00 | 4,23 |
| HPMC 603 | 1,00 | 0,85 |
| SDS | 0,25 | 0,21 |
| Acdisol | 12,00 | 10,15 |
| Avicel PH 101 | 100,00 | 84,57 |
| **Gesamtgewicht** | **118,25** | **100,00** |

Vermahlung in der Netsch Micro Cer (Nassvermahlung); Einstellungen:
Spaltsieb 0,1 mm; Mahlkörper 0,2-0,3 mm; Drehzahl Rührwerk 3000 U/min; Mahlraumdruck 0,3 bar

### Beispiel 3 (Formulierung 3):

| **Inhaltsstoff** | **mg/Tablette** | **Gew.-%** |
|---|---|---|
| Prasugrel Base | 5,00 | 4,23 |
| HPMC 603 | 1,00 | 0,85 |
| SDS | 0,25 | 0,21 |
| Acdisol | 12,00 | 10,15 |
| Lactose Anhydrat | 100,00 | 84,57 |
| **Gesamtgewicht** | **118,25** | **100,00** |

### Vermahlung im Ultraturrax (Nassvermahlung)

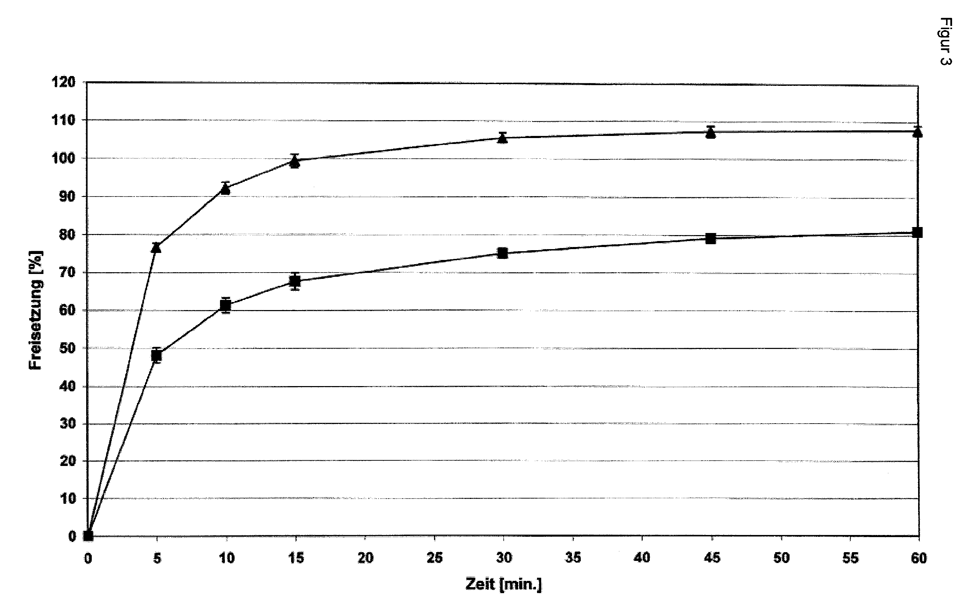

Das in Figur 3 gezeigte DSC der vermahlenen Zusammensetzung zeigt einen scharfen Peak bei 123°C und belegt damit, dass der mikronisierte Wirkstoff in kristalliner Form vorliegt.

### Beispiel 4 (Formulierung 4):

| **Inhaltsstoff** | **mg/Tablette** | **Gew.-%** |
|---|---|---|
| Prasugrel Base | 5,00 | 4,22 |
| HPMC 603 | 1,00 | 0,84 |
| SDS | 0,25 | 0,21 |
| Vitamin E Mischung | 0,20 | 0,17 |
| Acdisol | 12,00 | 10,13 |
| Avicel PH 101 | 100,00 | 84,42 |
| **Gesamtgewicht** | **118,45** | **100,00** |

### Vermahlung im Ultraturrax (Nassvermahlung)

### Beispiel 5 (Formulierung 5):

| **Inhaltsstoff** | **mg/Tablette** | **Gew.-%** |
|---|---|---|
| Prasugrel Base | 5,00 | 4,23 |
| Kollidon 30 | 1,00 | 0,85 |
| SDS | 0,25 | 0,21 |
| Acdisol | 12,00 | 10,15 |
| Avicel PH 101 | 100,00 | 84,57 |
| **Gesamtgewicht** | **118,25** | **100,00** |

### Vermahlung im Ultraturrax (Nassvermahlung)

### Beispiel 6 (Formulierung 6):

| **Inhaltsstoff** | **mg/Tablette** | **Gew.-%** |
|---|---|---|
| Prasugrel Base | 5,00 | 4,23 |
| HPMC 603 | 1,00 | 0,85 |
| SDS | 0,25 | 0,21 |
| Acdisol | 12,00 | 10,15 |
| Avicel PH 101 | 100,00 | 84,57 |
| **Gesamtgewicht** | **118,25** | **100,00** |

### Trockenvermahlung mit der Jetmill

### Beispiel 7 (Formulierung 7):

| **Inhaltsstoff** | **mg/Tablette** | **Gew.-%** |
|---|---|---|
| Prasugrel Base | 5,00 | 4,23 |
| Kollidon VA64 | 1,00 | 0,85 |
| SDS | 0,25 | 0,21 |
| Acdisol | 12,00 | 10,15 |
| Avicel PH 101 | 100, 00 | 84,57 |
| **Gesamtgewicht** | **118,25** | **100,00** |

Trockenvermahlung mit der Jetmill

### Beispiel 8 (Formulierung 8):

| **Inhaltsstoff** | **mg/Tablette** | **Gew.-%** |
|---|---|---|
| Prasugrel Base | 5,00 | 4,24 |
| Kollidon VA64 | 1,00 | 0,85 |
| Acdisol | 12,00 | 10,17 |
| Avicel PH 101 | 100, 00 | 84,75 |
| **Gesamtgewicht** | **118,00** | **100,00** |

Sprühtrocknung & Vermahlung im Ultra Turrax

In allen Beispielen wurde die Partikelgrößenverteilung nach der Vermahlung wie folgt gemessen:

| | |
|---|---|
| Instrument | Malvern Mastersizer 2000 mit Hydro S |
| Messzeit | 10 sec. |
| Anzahl der Proben | 3 |
| Hintergrund | 10 sec. |
| Model | Fraunhofer |
| Verdunkelungen | ca. 5 |
| Rührrate | 2500 UpM |
| Ultraschallbehandlung | 50 % |
| Ultraschallzeit | 60 sec. |
| Probenvorbereitung | 50 mg in HPLC Wasser + ein Tropfen Tween 80; 5 Minuten Ultraschallbehandlung |

Folgende Partikelgrößenverteilungen wurden gemessen:
Bei Vermahlen mit dem Ultraturrax (Nassvermahlung):
   D90: 15,7 µm D50: 3,4 µm
Bei Vermahlen mit der Netsch-Mühle (Nassvermahlung):
   D90: 0,2 mm
   D50: 0,15 µm
Bei Vermahlen mit der Jetmill (Trockenvermahlung):
   D90: 6,1 µm
   D50: 2,2 µm

### Bespiel 9: Stabilitätsstudien

### Wassergehalt (nach Karl-Fischer)

| | **Anfang** | **2 Wochen** | **4 Wochen** |
|---|---|---|---|
| **Formulierung 3** | 1,30 | 0, 90 | 1,00 |
| **Efient** | 1,50 | 1,60 | 2,00 |

Zersetzung (in %):

| | **Anfang** | **2 Wochen** | **4 Wochen** | **8 Wochen** |
|---|---|---|---|---|
| **Efient** | 0,47 | 0,51 | 0, 55 | 0,78 |
| **Formulierung 7** | 0,28 | 0,33 | 0, 50 | 0,67 |
| **Formulierung 3** | 0,42 | 0,53 | 0,53 | 0,72 |

### Beispiel 10: Freisetzung

Die Freisetzungsprofile wurden in 900 ml 50 mM Acetatpuffer (pH 4,5) bei 37°C und 75 UpM nach der USP Methode (App. II) gemessen.

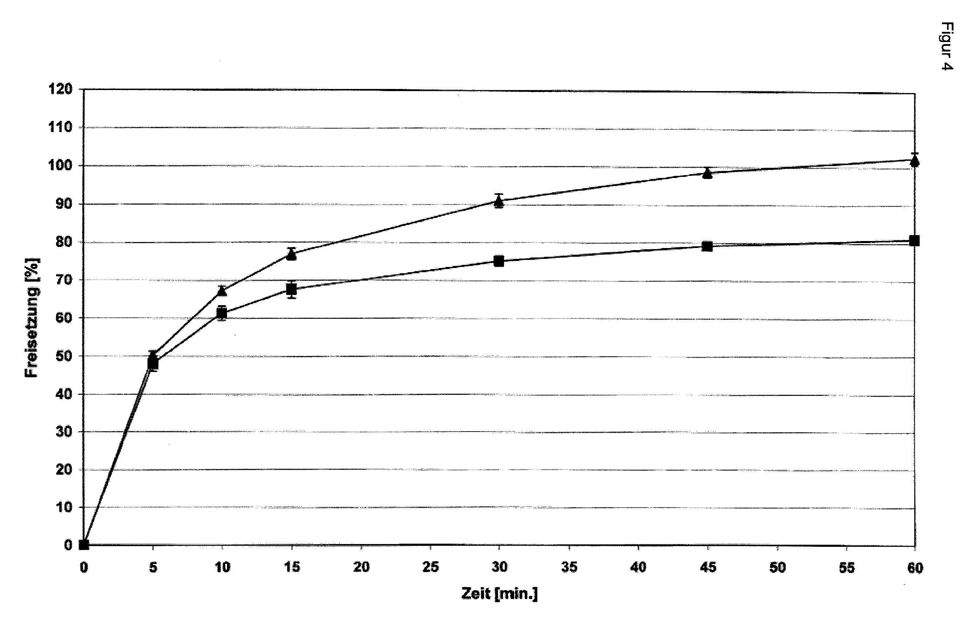

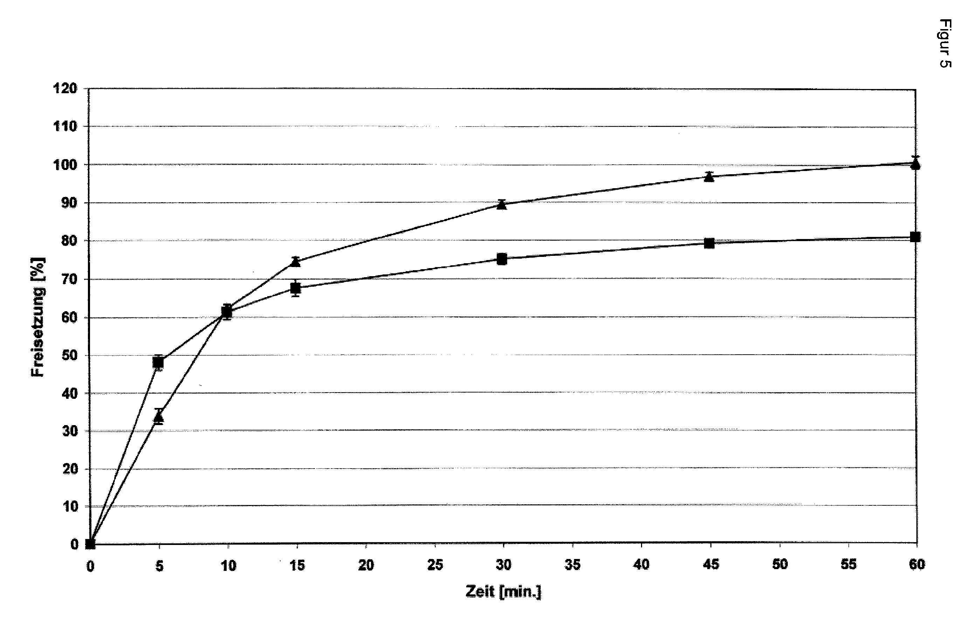

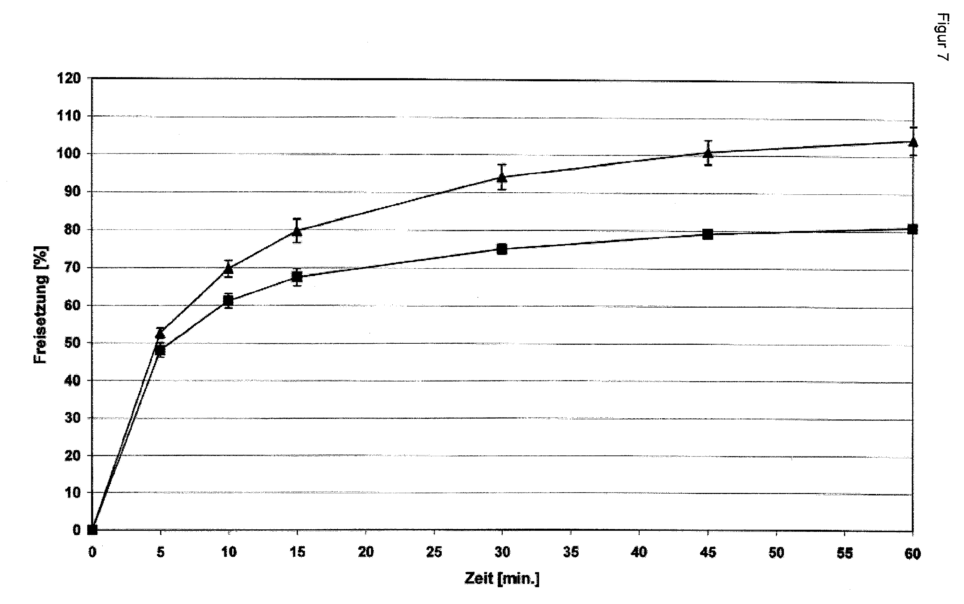

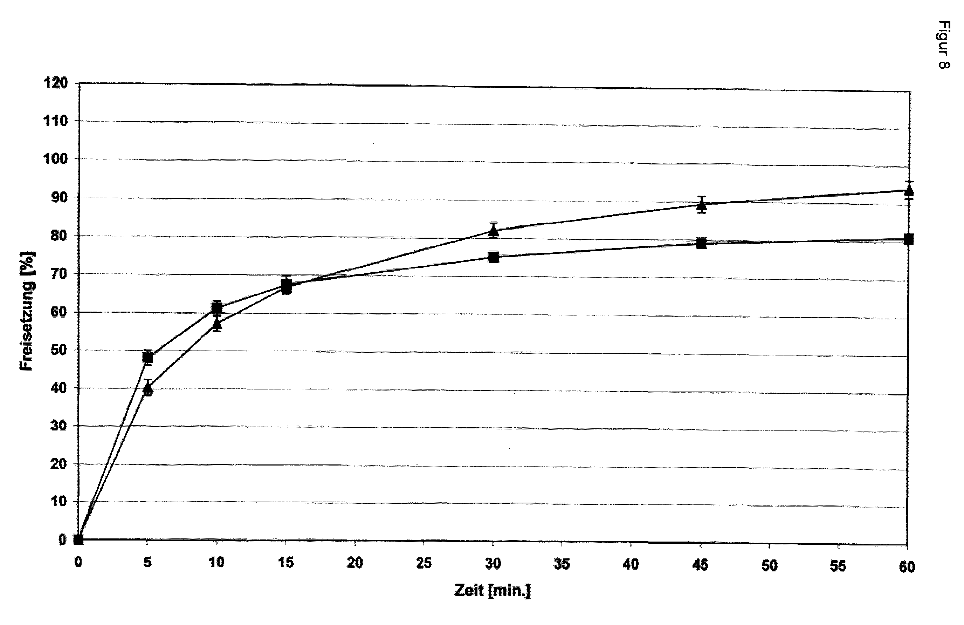

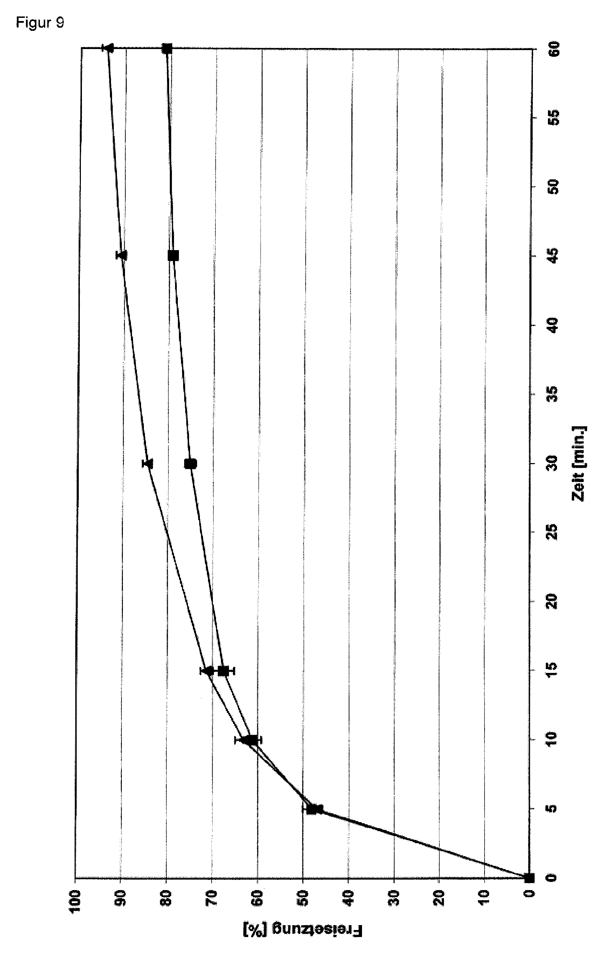

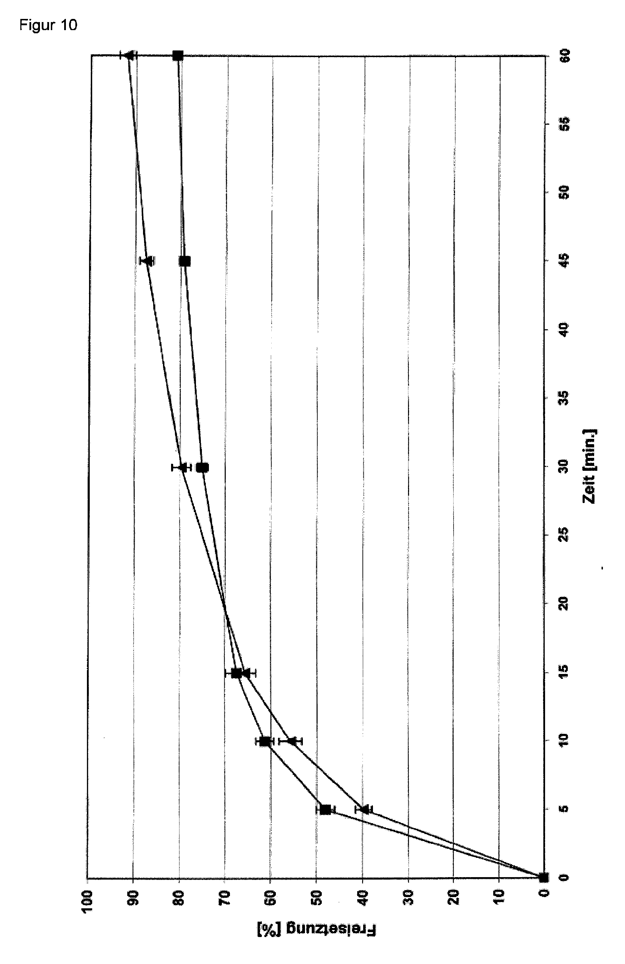

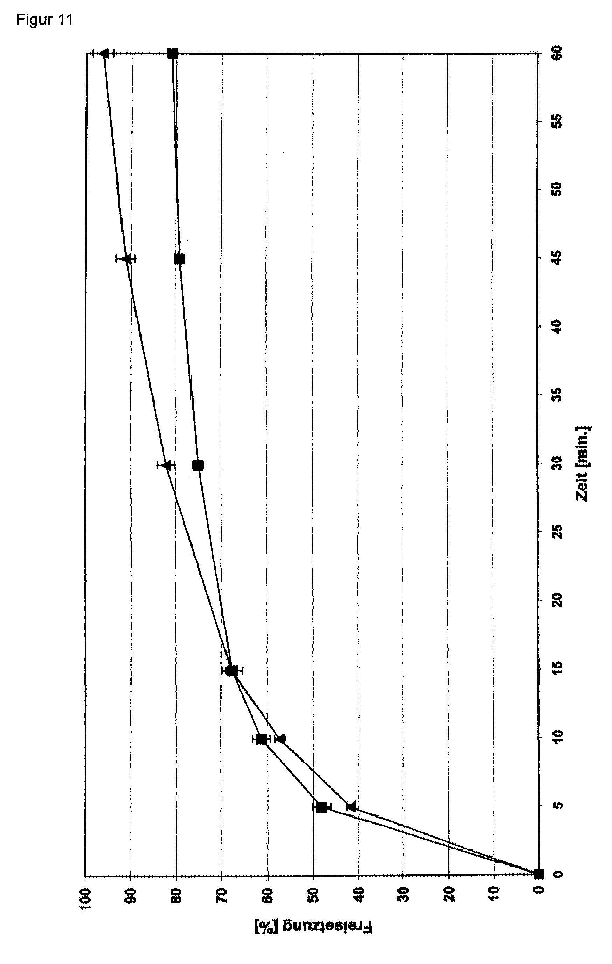

Die Freisetzungsprofile der Formulierungen der Beispiele 1-8 sind in den Figuren 3-5 und 7-11 jeweils zusammen mit dem Freisetzungsprofil des kommerziellen Prasugrel-Produkts Efient^{®} dargestellt. Man erkennt, dass die erfindungsgemäßen Zusammensetzungen im Vergleich zu dem kommerziellen Prasugrel-Produkt eine schnellere Freisetzung des Wirkstoffs zur Verfügung stellen. Zudem zeigt sich aus den Beispielen 9 und 10, dass die Hygroskopie der pharmazeutischen Zusammensetzung und auch der Wirkstoffabbau in der pharmazeutischen Formulierung im Vergleich zum kommerziellen Prasugrel-Produkt reduziert ist.

## Patentansprüche

1. Prasugrel oder ein pharmazeutisch verträgliches Salz davon in mikronisierter, kristalliner Form.

2. Prasugrel oder ein pharmazeutisch verträgliches Salz davon gemäß Anspruch 1, das einen Volumen-gewichteten mittleren Teilchendurchmesser D50 im Bereich von 0,1 bis 10 µm aufweist.

3. Prasugrel oder ein pharmazeutisch verträgliches Salz davon gemäß Anspruch 2, das einen Volumen-gewichteten Teilchendurchmesser D90 im Bereich von 0,2 bis 20 µm aufweist.

4. Zusammensetzung, enthaltend Prasugrel oder ein pharmazeutisch verträgliches Salz davon gemäß einem der Ansprüche 1-3 und ein hydrophiles Polymer.

5. Zusammensetzung gemäß Anspruch 4, wobei das Gewichtsverhältnis von Prasugrel oder einem pharmazeutisch verträglichen Salz davon, bezogen auf die freie Base des Prasugrels, zu hydrophilem Polymer > 1:4 ist.

6. Zusammensetzung gemäß Anspruch 4 oder 5, worin das hydrophile Polymer ausgewählt ist aus der Gruppe, bestehend aus Cellulosederivaten, wie Hydroxypropylmethylcellulose, Hydroxypropylcellulose, Carboxymethylcellulose, bevorzugt als Natrium- oder Calciumsalz, Hydroxyethylcellulose, Polyvinylpyrrolidon, Copolymeren von Polyvinylpyrrolidon, bevorzugt Copolymere umfassend Vinylpyrrolidon- und Vinylacetateinheiten, Polyoxyethylen-Alkylether, Polyethylenglycol, Co-Block-Polymeren von Ethylenoxid und Propylenoxid, Polymetacrylatderivaten, Polyvinylalkohol, Polyvinylderivaten und Polyethylenglycolderivaten.

7. Verfahren zur Herstellung einer Zusammensetzung gemäß einem der Ansprüche 4-6, umfassend die gemeinsame Vermahlung von Prasugrel oder einem pharmazeutisch verträglichen Salz davon und dem hydrophilen Polymer.

8. Verfahren gemäß Anspruch 7, worin die Vermahlung als Nass- oder Trockenvermahlung erfolgt.

9. Verfahren gemäß Anspruch 8, worin die Trockenvermahlung unter Kühlung erfolgt.

10. Verfahren gemäß Anspruch 7 oder 8, worin die Trockenvermahlung in einer Luftstrahlmühle erfolgt.

11. Verfahren gemäß Anspruch 10, worin die Trockenvermahlung in einer Luftstrahlmühle für einen Zeitraum von mindestens 30 Minuten, vorzugsweise mindestens einer Stunde erfolgt.

12. Zusammensetzung, erhältlich gemäß dem Verfahren nach einem der Ansprüche 7-11.

13. Pharmazeutische Zusammensetzung, enthaltend Prasugrel oder ein pharmazeutisch verträgliches Salz davon gemäß einem der Ansprüche 1-3 oder eine Zusammensetzung gemäß einem der Ansprüche 4-6 oder 12 und einen oder mehrere pharmazeutisch verträgliche Hilfsstoffe.

14. Verwendung von Prasugrel gemäß einem der Ansprüche 1-3 oder einer Zusammensetzung gemäß einem der Ansprüche 4-6 oder 12 zur Herstellung einer pharmazeutischen Zusammensetzung zur Plättchenaggregationshemmung.
